**Europäisches Patentamt**

**European Patent Office**

**Office européen des brevets**

(19)

(11) Numéro de publication : **0 375 763 B1**

(12) # FASCICULE DE BREVET EUROPEEN

(45) Date de publication du fascicule du brevet :
**28.10.92 Bulletin 92/44**

(21) Numéro de dépôt : **89906701.1**

(22) Date de dépôt : **22.06.89**

(86) Numéro de dépôt international :
**PCT/CH89/00120**

(87) Numéro de publication internationale :
**WO 89/12480 28.12.89 Gazette 89/30**

(51) Int. Cl.⁵ : **A61M 35/00, G01F 11/08**

(54) **DISPOSITIF APPLICATEUR POUR DEPOSER LOCALEMENT UNE SUBSTANCE LIQUIDE SUR UNE SURFACE A TRAITER.**

(30) Priorité : **23.06.88 FR 8808628**

(43) Date de publication de la demande :
**04.07.90 Bulletin 90/27**

(45) Mention de la délivrance du brevet :
**28.10.92 Bulletin 92/44**

(84) Etats contractants désignés :
**AT BE CH DE FR GB IT LI LU NL SE**

(56) Documents cités :
**FR-A- 1 288 915**
**FR-A- 1 314 002**
**FR-A- 1 385 377**
**GB-A- 1 588 204**
**US-A- 2 250 467**

(73) Titulaire : **GAFNER, Paul-Félix**
**La-Haut**
**CH-1435 Essert-Pittet (CH)**

(72) Inventeur : **GAFNER, Paul-Félix**
**La-Haut**
**CH-1435 Essert-Pittet (CH)**

(74) Mandataire : **Nithardt, Roland**
**Cabinet Roland Nithardt Conseils en Propriété**
**Industrielle S.A. Y-Parc Scientifique et**
**Technologique Chemin de la Sallaz**
**Casepostale 3347**
**CH-1400 Yverdon-les-Bains (CH)**

## Description

La présente invention concerne un dispositif applicateur pour déposer localement une substance liquide sur une surface à traiter, notamment une solution médicamenteuse sur une surface cutanée pour un traitement dermatologique, ce dispositif comportant un élément tubulaire allongé comprenant une cavité intérieure, un mécanisme de dosage à vis micrométrique et un embout de distribution pourvu d'un conduit capillaire communiquant avec ladite cavité intérieure, le mécanisme de dosage à vis micrométrique étant agencé pour faire varier le volume de ladite cavité intérieure, ladite cavité intérieure contenant au moins une enceinte contenant un volume déterminé de gaz, et le mécanisme de dosage à vis micrométrique comportant un élément mobile agencé pour comprimer plus ou moins ladite enceinte de manière à faire varier le volume de ladite cavité intérieure.

Le traitement dermatologique dit topique, au moyen de solutions médicamenteuses déposées à la surface d'une peau malade, s'effectue principalement au moyen de pinceaux, d'applicateurs en verre ou en matière synthétique. L'extrémité de ces applicateurs présente souvent un arrondi en forme de spatule en vue de déposer la solution de traitement sur la surface malade. Ces dispositifs sont parfois remplacés par des tubes de verre de faible diamètre comportant un conduit capillaire, formant une sorte de pipette.

Ces dispositifs connus se sont révélés inefficaces ou insuffisants pour le traitement des verrues, des kératoses actiniques ou séborrhéiques, des condylomes acuminés ou de certains cancers de la peau. En effet, avec ces dispositifs le médecin est pratiquement dans l'incapacité de doser avec précision les solutions thérapeutiques à utiliser, et de les appliquer sur le derme. De ce fait, ces moyens d'application se sont révélés d'une utilisation peu pratique et surtout imprécise.

Les applicateurs basés sur le principe de la microcapillarité présentent des inconvénients spécifiques en raison du fait qu'ils développent un film gras à l'intérieur du tube capillaire, ce qui exige un rinçage à l'alcool avant l'usage. De ce fait, ces applicateurs du type pipette s'avèrent compliqués, peu pratiques et nécessitent un temps de préparation relativement important avant toute opération d'application proprement dite.

Le brevet français N° 1 288 915 décrit un récipient à parois souples ouvert à une extrémité et contenant une substance liquide ou pâteuse, associé à un mécanisme agencé pour déformer ce récipient et provoquer l'écoulement de ladite substance par variation du volume du récipient. La substance contenue est directement en contact avec les parois intérieures de ce récipient qui ne peut en aucun cas être utilisé comme applicateur cutané de substances agressives sur les surfaces cutanées ou les muqueuses d'un patient, en raison de problèmes d'hygiène et de risques de contamination.

La présente invention se propose de pallier ces différents inconvénients en réalisant un dispositif applicateur tel que mentionné ci-dessus qui est d'utilisation aisée, de conception et de construction simples et qui permet un dosage précis de la solution thérapeutique, ainsi qu'une grande précision dans la mise en place de cette solution sur la surface cutanée à traiter.

Dans ce but, le dispositif applicateur selon l'invention est défini comme en revendication 1.

Selon un mode de réalisation avantageux, ladite enceinte comporte au moins une poche souple à parois lisses.

Selon un autre mode de réalisation, ladite enceinte comporte au moins une poche souple à parois plissées en accordéon.

Ladite enceinte peut comporter une poche souple unique fermée.

Dans une forme de réalisation avantageuse, la poche souple unique fermée est disposée dans ladite cavité intérieure de l'élément tubulaire allongé. Elle peut également être disposée dans ledit embout de distribution.

Dans une autre forme de réalisation ladite enceinte comporte deux poches souples qui communiquent au moyen d'un conduit axial et les deux poches souples peuvent être logées dans ladite cavité intérieure de l'élément tubulaire allongé ou l'une des poches souples peut être logée dans ledit embout de distribution.

D'une façon avantageuse, ledit conduit axial est capillaire.

De préférence, l'embout de distribution est prérempli et contient la substance liquide à déposer localement sur la surface à traiter.

Dans toutes les formes de réalisation, le mécanisme micrométrique comporte un capuchon pourvu d'un filetage micrométrique en prise avec un filetage micrométrique lié à l'élément tubulaire et agencé pour prendre appui sur ladite enceinte et pour communiquer des variations de volume à l'air contenu dans l'embout de distribution.

Selon la forme de réalisation, ledit élément tubulaire est agencé pour prendre appui sur la poche souple unique ou sur une des deux poches souples constituant ladite enceinte.

La présente invention sera mieux comprise en référence à la description d'exemples de réalisation du dispositif applicateur selon l'invention et au dessin annexé, dans lequel :

la fig. 1 représente une vue en coupe axiale d'une forme de réalisation particulière du dispositif applicateur selon l'invention,

la fig. 2 représente une deuxième forme de réalisation du dispositif applicateur selon l'invention,

la fig. 3 représente une vue en coupe illustrant une autre forme de réalisation du dispositif selon l'invention,

les fig. 4 à 7 illustrent différentes variantes d'embouts de distribution adaptables à des dispositifs applicateurs tels que représentés par la fig. 3.

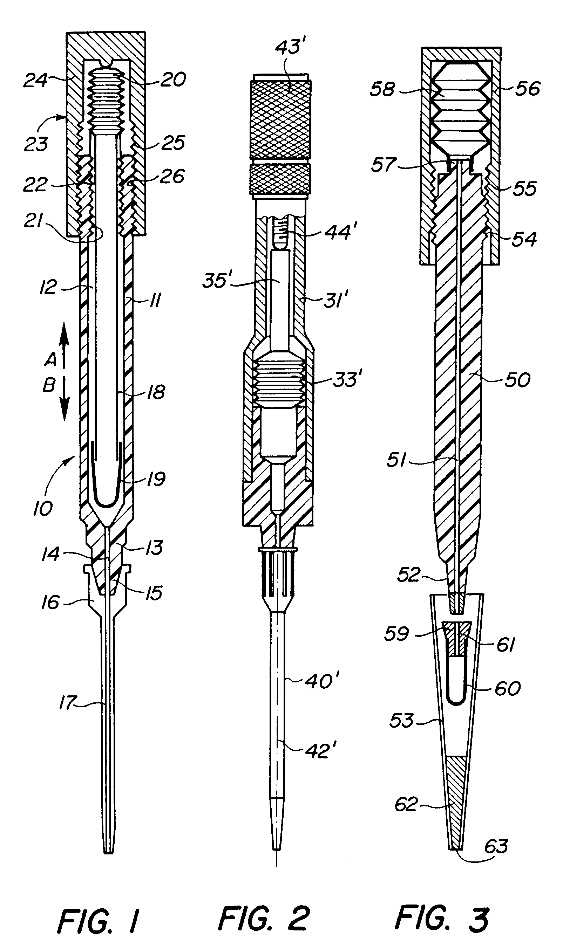

En référence à la fig. 1, le dispositif applicateur 10 représenté se compose principalement d'un élément tubulaire allongé 11, que l'on peut appeler le corps du dispositif et qui définit une cavité intérieure 12. A l'une de ses extrémités l'élément tubulaire 11 présente un section rétrécie 13 traversée axialement par un conduit capillaire 14. Ladite section rétrécie a la forme d'un cône de raccordement 15 permettant la mise en place d'un embout de distribution 16 également traversé par un conduit capillaire axial 17 qui est connecté au conduit capillaire 14 de la section rétrécie 13.

La cavité intérieure 12 de l'élément tubulaire 11 du dispositif applicateur 10 contient un tube 18 de forme cylindrique, obturé à son extrémité proche de l'embout de distribution 16 par une première poche souple 19 et à son extrémité opposée par une seconde poche souple 20 ayant la forme d'une tétine plissée en accordéon, qui peut être comprimée puis reprendre sa position initiale. L'ensemble ainsi formé constitue une enceinte 9. Afin de pouvoir modifier le volume de cette enceinte, le tube 18 est monté, par exemple par vissage grâce à un filetage périphérique 21, dans l'élément tubulaire 11 pourvu, dans ce but, d'un filetage intérieur 22. Par ailleurs, le dispositif comporte un mécanisme 23 à vis micrométrique. Ce mécanisme comporte en fait un capuchon 24 pourvu d'un filetage intérieur 25 qui est en prise avec un filetage extérieur 26 de l'élément tubulaire cylindrique 11. Les deux filetages 25 et 26 coopèrent l'un avec l'autre et ont un pas très faible de sorte qu'une rotation d'un nombre de tours relativement élevé du capuchon 24 engendre un déplacement axial relativement faible de ce capuchon. Ce déplacement a pour conséquence un appui et une compression plus ou moins forts de la poche 20.

Le capuchon est conçu pour agir dans les deux sens, d'une part "en montée", c'est-à-dire dans le sens illustré par la flèche A, ce qui provoque l'aspiration d'une certaine quantité de solution thérapeutique à travers le conduit capillaire 17, et d'autre part "en descente", dans le sens illustré par la flèche B, ce qui a pour effet de refouler une quantité déterminée de cette solution à travers le capillaire 17, et son dépôt sur la surface à traiter.

Ce dispositif se tient aisément à l'aide d'une main, et le capuchon 24 peut facilement être tourné dans un sens ou dans l'autre par les doigts de cette même main de l'opérateur. De ce fait, cet opérateur dispose librement de son autre main, ce qui est particulièrement important pour assurer une mise en place précise de la solution médicamenteuse en un endroit déterminé d'une surface cutanée. Grâce au mécanisme de dosage à vis micrométrique, la quantité de liquide refoulée à travers le conduit capillaire peut être contrôlée avec précision. On notera que le liquide n'est évacué que pendant que l'opérateur, tourne le capuchon et que cet écoulement est immédiatement arrêté lorsque l'opérateur cesse de tourner ce capuchon. Il peut de ce fait contrôler à vue, d'une manière extêmement précise, la quantité de liquide déposée sur la surface à traiter. Bien que l'une des applications essentielles de ce dispositif soit du domaine médical et thérapeutique, d'autres utilisations peuvent être envisagées. En particulier, le dépôt de colles spéciales pour le collage de pièces de mécanique ou de composants électroniques. Cette forme de réalisation est particulièrement avantageuse en ce qu'elle permet de séparer de façon effective la cavité intérieure de l'élément tubulaire de l'embout contenant les substances éventuellement agressives qui servent au traitement envisagé. La variation de volume intérieur se traduit par une aspiration ou un refoulement du liquide de traitement dans le conduit capillaire.

La fig. 2 illustre une autre forme de réalisation avantageuse d'un dispositif applicateur. Le principe de ce dispositif, les buts qu'il se propose d'atteindre et les résultats obtenus sont sensiblement les mêmes que ceux décrits ci-dessus en référence à la fig. 1. Ce dispositif applicateur cutané comporte également un élément tubulaire allongé 31' qui définit une cavité intérieure dans laquelle sont logés d'une part une poche compressible fermée 33' dont les parois sont repliées en forme d'accordéon, ce qui facilite sa compression et son retour dans sa position initiale, et d'autre par un organe d'appui 3'. Cet organe d'appui 35', de forme cylindrique, coulisse à l'intérieur de la cavité de l'élément tubulaire en fonction du déplacement axial d'une tige filetée 44', elle-même liée à un capuchon moleté 43'. Cet organe d'appui pourrait également être rendu solidaire de la tige filetée.

Dans cette réalisation, la poche 33' est compressible sous l'effet du déplacement de l'organe d'appui 35'. Un appui sur la poche 33' a pour effet d'augmenter le volume intérieur dans la cavité de l'élément allongé 31' et par conséquent, d'engendrer une aspiration du liquide dans le capillaire 42' de l'embout 40'.

La poche 33' peut être réalisée en une matière plastique qui ne rentre pas dans la catégorie des élastomères, etant donné que l'élasticité de cette poche est obtenue par le pliage en accordéon.

Le dispositif tel que représenté par la fig. 3 est une variante du dispositif illustré par la fig. 1. Comme dans cet exemple, il comporte un élément tubulaire allongé 50 traversé de part en part par un conduit 51 qui est de préférence un conduit capillaire. A proximité de l'une de ses extrémités, l'élément tubulaire 50 présente une section rétrécie sensiblement tronconique qui est adaptée pour recevoir un embout de distribution 53. Le tronçon d'extrémité opposée est équipé d'un filetage extérieur 54 qui est agencé pour être en prise avec le filetage intérieur 55 d'un capuchon moleté 56. Un embout 57 cylindrique de section rétrécie porte une poche souple 58 dont les parois sont de préférence plissées en forme de soufflet d'accordéon.

A l'intérieur de l'embout de distribution 53 est monté un élément composé d'un bouchon d'obturation 59 qui porte une deuxième poche souple 60. Le bouchon d'obturation 59 est percé d'un conduit capillaire 61 qui communique avec l'intérieur de la poche souple 60. Sur la figure, le bouchon d'obturation ne se trouve pas dans sa positon finale. En effet, dans cette position, le bouchon est en appui étanche contre les parois intérieures de l'embout de distribution 53. Par ailleurs, cet embout de distribution est engagé sur l'extrémité de l'élément tubulaire 50 de manière à rendre etanche la liaison entre ces deux éléments. Dans l'exemple représenté, l'embout de distribution 53 est du type prérempli et contient une substance de traitement, par exemple une substance médicamenteuse.

L'espace délimité par la poche souple 58, le conduit capillaire 51, le conduit capillaire 61 et la poche souple 60 définit un volume de référence dont la variation peut être obtenue par compression de la poche souple 58 sous l'effet d'une rotation du capuchon 56. La compression de la poche souple 58 se répercute au niveau de la poche souple 60, ce qui a pour effet de faire varier le volume d'air situé au-dessus du niveau de la substance liquide 62. Cette augmentation du volume a pour effet de refouler une certaine quantité de cette substance par une ouverture 63 ménagée à l'extrémité de cet embout. Lorsque l'embout est, comme le montre la figure, du type prérempli, l'utilisateur tourne le capuchon 56 dans un sens pour refouler la substance de traitement hors de cet embout. En revanche, lorsque l'embout n'est pas du type prérempli, c'est-à-dire lorsqu'une quantité de substance de traitement doit préalablement être prélevée dans un réservoir approprié, l'opérateur comprime tout d'abord la poche souple 58 puis la détend pour engendrer une aspiration de cette substance jusqu'à une hauteur déterminée de l'embout de distribution.

Comme mentionné précédemment, cette forme de réalisation est particulièrement avantageuse en ce qu'elle préserve l'intérieur du dispositif applicateur contre la pénétration de substances agressives ou de vapeurs de ces substances. Les embouts peuvent être conçus pour être jetés.

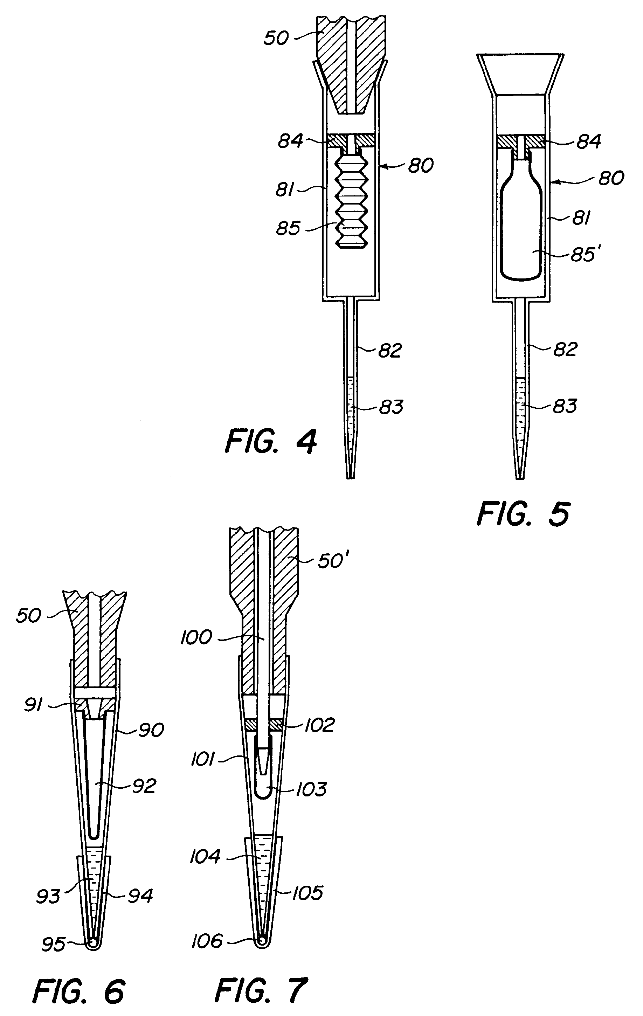

La fig. 4 illustre une autre forme de réalisation d'un embout adapté à un élément tubulaire 50 tel que représenté par la fig. 3. Dans cette variante, l'embout de distribution 80 se compose d'un élément supérieur sensiblement cylindrique 81 et d'un élément inférieur capillaire 82 qui contient une substance de traitement 83. L'élément supérieur 81 est obturé par un bouchon d'obturation 84 auquel est adaptée une poche souple 85 dont les parois sont plissées en forme de soufflet d'accordéon. Comme précédemment, les variations de volume engendrées par la poche supérieure 58 se répercutent sur la poche souple 85 et provoquent le déplacement dans un sens ou dans l'autre de la substance de traitement 83 contenue dans la pointe capillaire 82 de l'embout de distribution.

La fig. 5 représente une variante de l'embout illustré par la fig. 4. En fait, cette réalisation diffère par la forme de la poche 85' qui comporte des parois lisses.

La fig. 6 illustre une variante dans laquelle l'embout de distribution 90 contient un bouchon d'obturation 91 sur lequel est fixée une poche souple 92, à parois lisses, qui répercute les variations de volume engendrées par la compression de la poche souple supérieure 58 montée à l'extrémité supérieure (non représentée) de l'élément tubulaire allongé 50. Dans ce cas également, l'embout de distribution est du type prérempli et contient une substance de traitement 93. Cet embout est obturé par un capuchon 94 qui peut être engagé de force ou vissé sur la pointe d'extrémité de l'embout. Une bille 95 assure l'obturation de l'ouverture de cet embout.

La fig. 7 illustre une autre forme de réalisation dans laquelle l'élément tubulaire allongé 50' du dispositif applicateur contient un élément tubulaire 100 qui se prolonge vers l'intérieur de l'embout de distribution 101 à travers un organe d'obturation 102 et qui porte une poche souple 103. Cet embout est également du type prérempli et contient une substance de traitement 104. Il est obturé par un cône de fermeture 105 contenant une bille d'obturation 106.

La présente invention n'est pas limitée aux formes de réalisation décrites mais peut subir différentes modifications et se présenter sous diverses variantes évidentes pour l'homme de l'art. La forme et les dimensions des différents composants peuvent être adaptées aux conditions d'utilisation de l'applicateur.

## Revendications

1. Dispositif applicateur cutané pour déposer localement une substance liquide sur une surface à traiter, notamment une solution médicamenteuse sur une surface cutanée pour un traitement dermatologique, ce dispositif comportant un élément tubulaire allongé (11) comprenant une cavité intérieure (12), un mécanisme de dosage à vis micrométrique (23) et un embout de distribution (16) pourvu d'un conduit capillaire (19) communiquant avec ladite cavité intérieure, le mécanisme de dosage à vis micrometrique etant agencé pour faire varier le volume de ladite cavité intérieure, ladit cavité intérieure (12) contenant au moins une enceinte comprimable (9) contenant un volume determiné de gaz, et le mécanisme (23) de dosage à vis micrométrique comportant un élément mobile agencé pour comprimer plus ou moins ladite enceinte comprimable (9) de manière à faire varier le volume de ladite cavité intérieure, caractérisé en ce que ladite enceinte comprimable (9) est fermée et comporte au moins une poche souple (19,20).

2. Dispositif selon la revendication 1, caractérisé en ce que ladite enceinte (9) comporte au moins une poche souple à parois lisses (19).

3. Dispositif selon la revendication 1, caractérisé en ce que ladite enceinte (9) comporte au moins une poche souple à parois plissées en accordéon (20, 33', 58).

4. Dispositif selon la revendication 1, caractérisé en ce que ladite enceinte (9) comporte une poche souple unique fermée (33', 58).

5. Dispositif selon la revendication 4, caractérisé en ce que la poche souple unique fermée (33') est disposée dans ladite cavité intérieure de l'élément tubulaire allongé (31').

6. Dispositif selon la revendication 4, caractérisé en ce que la poche souple unique fermée (60) est disposée dans ledit embout de distribution (53).

7. Dispositif selon la revendication 1, caractérisé en ce que ladite enceinte comporte deux poches souples (19, 20) qui communiquent au moyen d'un conduit axial (18).

8. Dispositif selon la revendication 7, caractérisé en ce que les deux poches souples (19, 20) sont logées dans ladite cavité intérieure (12) de l'élément tubulaire allongé (11).

9. Dispositif selon la revendication 7, caractérisé en ce que l'une des poches souples (60, 85, 85', 92, 103) est logée dans ledit embout de distribution (53, 80, 90, 101).

10. Dispositif selon la revendication 7, caractérisé en ce que ledit conduit axial (51) est capillaire.

11. Dispositif selon la revendication 1, caractérisé en ce que l'embout de distribution (53) est prérempli et contient la substance liquide à déposer localement sur la surface à traiter.

12. Dispositif selon la revendication 1, caractérisé en ce que le mécanisme micrométrique (23) comporte un capuchon (24) pourvu d'un filetage micrométrique (25) en prise avec un filetage micrométrique (26) lié à l'élément tubulaire (11) et agencé pour prendre appui sur ladite enceinte (9) et pour communiquer des variations de volume à l'air contenu dans l'embout de distribution (16).

13. Dispositif selon la revendication 12, caractérisé en ce que ledit élément tubulaire (11) est agencé pour prendre appui sur la poche souple unique ou sur une des deux poches souples constituant ladite enceinte (9).

**Claims**

1. Cutaneous applicator device for locally depositing a liquid substance onto a surface to be treated, particularly a medicinal solution onto a cutaneous surface for a dermatological treatment, this device having an elongated tubular element (11) comprising an internal cavity (12), a micrometric-screw dosing mechanism (23), and a distribution tip (16) provided with a capillary conduit (17) communicating with the said internal cavity, the micrometric-screw dosing mechanism being designed to allow the variation of the volume of the said internal cavity, the said internal cavity (12) containing at least one compressible chamber (9) containing a determined volume of gas, and the micrometric-screw dosing mechanism (23) comprising at least a mobile element designed to compress more or less said compressible chamber (9) so as to allow the variation of the volume of the said internal cavity, characterized in that the said compressible chamber (9) is closed and comprises at least a supple pocket (19, 20).

2. Device according to claim 1, characterized in that the said chamber comprises at least a smooth-walled supple pocket (19).

3. Device according to claim 1, characterized in that the said chamber (9) comprises at least a supple pocket with its walls pleated like an accordion (20, 33', 58).

4. Device according to claim 1, characterized in that the said chamber (9) comprises a single closed supple pocket (33', 58) .

5. Device according to claim 4, characterized in that the single closed supple pocket (33') is lodged in the internal cavity of the elongated tubular element (31') .

6. Device according to claim 4, characterized in that the single closed supple pocket (60) is lodged in the said distribution tip (53).

7. Device according to claim 1, characterized in that the said chamber comprises two supple pockets (19, 20) which are in communication by means of an axial conduit (18).

8. Device according to claim 7, characterized in that the two supple pockets (19, 20) are lodged in the said internal cavity (12) of the elongated tubular element (11).

9. Device according to claim 7, characterized in that one of the supple pockets (60, 85, 85', 92, 103) is lodged in the said distribution tip (53, 80, 90, 101).

10. Device according to claim 7, characterized in that the said axial conduit (51) is capillary.

11. Device according to claim 1, characterized in that the distribution tip (53) is prefilled and contains the liquid substance to be deposited locally on the surface to be treated.

12. Device according to claim 1, characterized in that the micrometric mechanism (23) comprises a cap (24) provided with a micrometric-screw (25) engaged with a micrometric thread (26) linked to the tubular element (11) and designed to press against the said chamber (9) and to communicate variations in the volume of air contained in the distribution tip (16).

13. Device according to claim 12, characterized in that the tubular element (11) is designed to press against the single supple pocket or against one of the two supple pockets constituting the said chamber (9).

**Patentansprüche**

1. Vorrichtung zur Verwendung auf der Haut, um örtlich eine flüssige Substanz auf eine zu behandeln de Fläche aufzutragen, insbesondere eine heilende Lösung auf eine Hautfläche für eine Hautbehand lung, wobei diese Vorrichtung aus folgendem besteht: einem länglichen rohrförmigen Teil (11), das eine innere Kammer (12) aufweist, einem Me chanismus zur Dosierung mittels einer Mikrometerschraube (23) und einem Auftragestutzen (16), der mit einer Kapillarbohrung (19) versehen ist, wel che mit der inneren Kammer in Verbindung steht, wobei der Mechanismus zur Dosierung mittels Mikro meterschraube so ausgeführt ist, daß man das Volu men der inneren Kammer variieren kann, und die innere Kammer (12) mindestens einen komprimierbaren Raum (9) umschließt, der ein bestimmtes Gasvolumen enthält, und weiterhin der Mechanismus (23) zur Dosierung mittels Mikrometerschraube ein bewegliches Teil aufweist, welches dazu dient, den komprimierbaren Raum (9) mehr oder weniger zu komprimieren, um das Volumen der inneren Kammer variieren zu lassen,
**dadurch gekennzeichnet, daß**
der komprimierbare Raum (9) geschlossen ist und mindestens einen flexiblen Behälter (19,20) aufweist.

2. Vorrichtung nach Anspruch 1,
**dadurch gekennzeichnet, daß**
der Raum (9) mindestens einen flexiblen Behälter mit glatten Wänden (19) aufweist.

3. Vorrichtung nach Anspruch 1,
**dadurch gekennzeichnet, daß**
der Raum (9) mindestens einen flexiblen Behälter mit zieharmonikaartig gefältelter Wand (20,33',58) aufweist.

4. Vorrichtung nach Anspruch 1,
**dadurch gekennzeichnet, daß**
der Raum (9) einen einzigen flexiblen und geschlossenen Behälter (33',58) aufweist.

5. Vorrichtung nach Anspruch 4,
**dadurch gekennzeichnet, daß**
der einzige flexible und geschlossene Behälter (33') in der inneren Kammer des länglichen rohrförmigen Teils (31') angeordnet ist.

6. Vorrichtung nach Anspruch 4,
**dadurch gekennzeichnet, daß**
der einzige flexible und geschlossene Behälter (60) in dem Auftragestutzen (53) angeordnet ist.

7. Vorrichtung nach Anspruch 1,
**dadurch gekennzeichnet, daß**
der Raum zwei flexible Behälter (19,20) aufweist, die über eine axiale Bohrung (18) miteinander verbunden sind.

8. Vorrichtung nach Anspruch 7,
**dadurch gekennzeichnet, daß**
die zwei flexiblen Behälter (19,20) in der inneren Kammer (12) des länglichen rohrförmigen Teiles (11) untergebracht sind.

9. Vorrichtung nach Anspruch 7,
**dadurch gekennzeichnet, daß**
einer der flexiblen Behälter (60,85,85',92,103) in dem Auftragestutzen (53,80,90,101) untergebracht ist.

10. Vorrichtung nach Anspruch 7,
**dadurch gekennzeichnet, daß**
die axiale Bohrung (51) als Kapillare ausgeführt ist.

11. Vorrichtung nach Anspruch 1,
    **dadurch gekennzeichnet,** daß
    der Auftragestutzen (53) vorgefüllt ist und die lokal auf der zu behandelnden Fläche aufzutragende flüssige Substanz enthält.

12. Vorrichtung nach Anspruch 1,
    **dadurch gekennzeichnet,** daß
    der Mikrometermechanismus (23) eine mit Mikrometergewinde (25) versehene Kappe (24) aufweist, die auf einem, an dem rohrförmigen Teil (11) befindlichen Mikrometergewinde (26) sitzt und dazu dient, einen Druck auf den Raum (9) auszuüben und um Volumenänderungen auf die in den Auftragestutzen (16) enthaltene Luft zu übertragen.

13. Vorrichtung nach Anspruch 12,
    **dadurch gekennzeichnet,** daß
    das rohrförmige Teil (11) so ausgeführt ist, daß es auf den einzelnen flexiblen oder auf einen der zwei flexiblen Behälter, die den Raum (9) darstellen, als Abstützung wirkt.

FIG. 1

FIG. 2

FIG. 3

FIG. 4

FIG. 5

FIG. 6

FIG. 7